# EUROPÄISCHE PATENTSCHRIFT

(11) **EP 1 296 634 B1**
(45) Veröffentlichungstag und Bekanntmachung des Hinweises auf die Patenterteilung: **21.09.2005**
(21) Anmeldenummer: 01957913.5
(22) Anmeldetag: 03.07.2001
(51) Int. Cl.: A61K 6/083, C07F 9/38

(54) **PHOSPHONSÄUREN ENTHALTENDES DENTALMATERIAL**
DENTAL MATERIAL CONTAINING PHOSPHONIC ACIDS
PRODUIT DENTAIRE RENFERMANT DE L'ACIDE PHOSPHONIQUE

(30) Priorität: 06.07.2000 EP 00114527
(43) Veröffentlichungstag der Anmeldung: 02.04.2003
(73) Patentinhaber: Ernst Mühlbauer GmbH & Co.KG, 25870 Norderfriedrichskoog (DE)
(72) Erfinder: ERDMANN, Christoph, 71334 Waiblingen (DE); ZIEGLER, Silke, 22049 Hamburg (DE); NEFFGEN, Stephan, 22459 Hamburg (DE); BOLLN, Carsten, 78315 Radolfzell (DE); MÜHLBAUER, Wolfgang, 22609 Hamburg (DE); LÜCK, Rainer, 25436 Tornesch (DE)
(74) Vertreter: Glawe, Delfs, Moll
(86) Internationale Anmeldenummer: PCT/EP2001/007602
(87) Internationale Veröffentlichungsnummer: WO 2002/002057

(56) Entgegenhaltungen:
- EP-A- 0 089 654
- EP-A- 0 909 761
- DE-A- 19 918 974
- IGNATIOUS F: "NOVEL CARBAMOYL PHOSPHONATE MONOMERS AND POLYMERS FROM UNSATURATED ISOCYANATES" JOURNAL OF POLYMER SCIENCE, POLYMER CHEMISTRY EDITION,US,JOHN WILEY AND SONS. NEW YORK, Bd. 31, Nr. 1, 1993, Seiten 239-247, XP000331769 ISSN: 0887-624X in der Anmeldung erwähnt

## Beschreibung

Die Erfindung betrifft Dentalmaterialien, insbesondere Haftvermittler oder polymerisierbare Zemente wie bspw. Compomere oder Glasionomerzemente mit polymerisierbaren Säuren, wie sie grundsätzlich aus DE-A-3536076 und DE-A-3536077 bekannt sind.

Dentalmaterialien mit polymerisierbaren Phosphorsäuren als Comonomere sind bekannt, sie enthalten üblicherweise Phosphorsäure und Acrylate oder Methacrylate. Aus DE-A-19647140 ist es bspw. bekannt, ein Hydroxyalkylacrylat bzw. -methacrylat mit Phosphat zu verestern. Nachteilig an diesen Substanzen ist die geringe Hydrolysestabilität, da die Esterbindungen zwischen Phosphat und Alkylkette sowie zwischen Methacrylat und Alkylkette leicht hydrolytisch gespalten werden können. Dies vermindert die Lagerstabilität des Dentalmaterials und führt unter den Bedingungen in der Mundhöhle zu einer reduzierten Dauerhaltbarkeit im Zahn.

Aus DE-A-19746708 ist es bekannt, Phosphonate über einen Spacer an die Methylgruppe eines Methacrylatesters zu binden. Bekannt sind ferner Phosphinoxide, die über eine Urethangruppe mit Methacrylaten oder Styrol verknüpft sind (J. Smid et al., Journal of Polymer Science, Part A Polymer Chemistry, 31, 239 - 247 (1993)) sowie die Verknüpfung von Phosphonsäuren mit Methacrylaten über einen Ester (DE-A-19918974). Der Erfindung liegt die Aufgabe zugrunde, Dentalmaterialien der eingangs genannten Art zu schaffen, die gute Hafteigenschaften und eine hohe Hydrolysestabilität aufweisen.

Die Erfindung löst diese Aufgabe durch die Merkmale des Hauptanspruchs. Dementsprechend enthalten die Dentalmaterialien Phosphonsäuren der nachfolgend angegebenen Struktur bzw. Salze dieser Säuren: 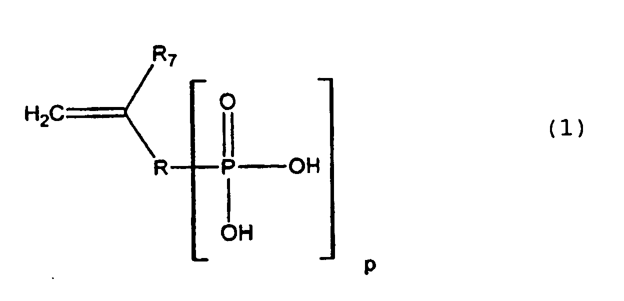 wobei R, R₇ und p unabhängig voneinander bedeuten:
entweder:
   a) R: Alkylgruppe oder Alkylengruppe mit mindestens 6 C-Atomen oder Arylgruppe,
      R₇: H, Methyl, Ethyl, Propyl, Isopropyl oder Butyl, p gleich 1 oder 2;
      oder:
   b) R: -CO-NR₁-R₈-
      mit R₁ gleich H, Alkyl oder Aryl,
      mit R₈ gleich Aryl oder CₙH₂ₙ
      wobei 4 ≤ n ≤ 18 ist,
      oder R₈ gleich CₙH₂ₙ-Si(R₅)₂-[O-Si(R₅)₂]ₘ-CₙH₂ₙ-
      wobei 3 ≤ n ≤ 12,
      1 ≤ m ≤ 10, und
      R₅ gleich Methyl, Ethyl oder Phenyl ist,
      oder R₈ gleich CₙH₂ₙ-COONH-CₙH₂ₙ
      wobei 4 ≤ n ≤ 12 ist, und wobei R₈ Ether- oder weitere Urethangruppen aufweisen kann,
      R₇ und p wie in a) definiert sind;
      oder:
c) R: 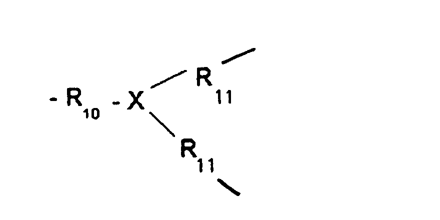 mit R₁₀ ausgewählt aus der Gruppe bestehend aus Arylgruppen, Alkylgruppen mit mindestens 3 C-Atomen, oder Polyethergruppen mit 1 bis 10 Polyethereinheiten, mit R₁₁ gleich oder verschieden ausgewählt aus der Gruppe bestehend aus Alkylgruppen und Arylgruppen,
   mit X gleich N, B oder CH,
   R₇: COOR₉, CONHR₉, H oder Phenyl mit R₉ ausgewählt aus der Gruppe bestehend aus Methyl, Ethyl, Propyl, Isopropyl und Butyl,
   p gleich 2.

Der Erfindung liegt die überraschende Erkenntnis zugrunde, daß Moleküle mit einer verhältnismäßig langkettigen Brücke zwischen Phosphonsäuregruppe und reaktiver Doppelbindung, wie sie im Anspruch definiert ist, eine hohe Hydrolysestabilität und gleichzeitig verbesserte Hafteigenschaften aufweisen.

Bevorzugte Ausgestaltungen der Erfindung sind in den Unteransprüchen angegeben.

Eine bevorzugte Ausführungsform der Erfindung hat die nachfolgend angegebene Struktur:

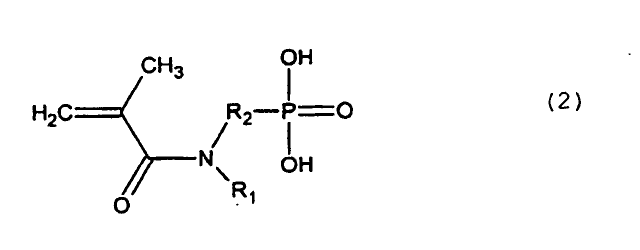

R₁ ist Wasserstoff, eine Alkyl- oder Arylgruppe. Bevorzugt wird R₁ ausgewählt aus der Gruppe bestehend aus Wasserstoff, Methyl, Ethyl und Isopropyl. R₂ ist eine Alkylgruppe mit 4 bis 18 C-Atomen, die unverzweigt oder verzweigt sein kann. Bevorzugt enthält sie 6 bis 12 C-Atome. Im Rahmen der Erfindung kann R₂ auch als Alkylkette ausgebildet sein, die durch Ethergruppen oder weitere Urethangruppen unterbrochen ist.

Bei einer weiteren bevorzugten Ausführungsform haben die Phosphonsäuren folgende Struktur:

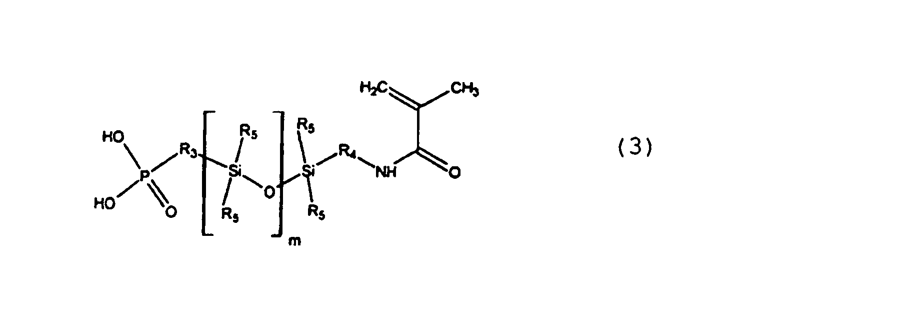

In dieser Formel sind R₃ oder R₄ Aryl- oder Alkylgruppen. Die Alkylgruppen können unverzweigt oder verzweigt sein und weisen vorzugsweise 3 bis 12 C-Atome auf. Die Substituenten R₅ sind gleich oder verschieden und stellen Methyl-, Ethyl- oder Phenylgruppen dar. m ist gleich 1 bis 10.

Unter den Phosphonsäuren mit Silikonanteilen in der Brückenverbindung zwischen reaktionsfähiger Doppelbindung und Phosphonsäure ist folgende Struktur bevorzugt:

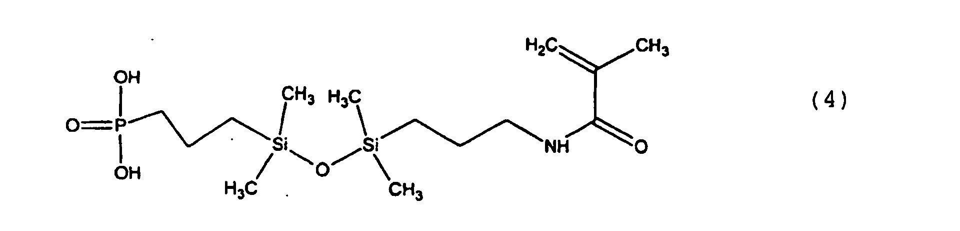

Folgende Diphosphonate sind bevorzugt in den erfindungsgemäßen Dentalmaterialien verwendbar:

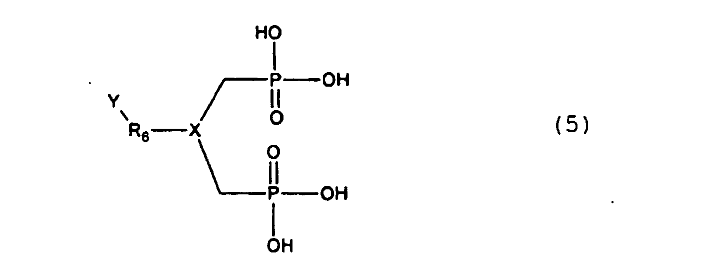

X ist Stickstoff, Bor oder eine CH-Gruppe, R₆ ist eine unverzweigte oder verzweigte Alkylgruppe mit wenigstens 3 C-Atomen, eine Arylgruppe oder eine Polyethergruppe mit 1 bis 10 Polyethereinheiten, und Y enthält ethylenisch ungesättigte Doppelbindungen, insbesondere ist Y ausgewählt aus der Gruppe bestehend aus CH₂=CH-O-, Styrol, Methacrylamid und CH₂=C(COOR₁₂)-CH₂-, wobei R₁₂ gleich H, Methyl, Ethyl, Propyl, iso-Propyl oder Butyl ist.

Gemäß einer bevorzugten Ausführungsform der Erfindung enthält das erfindungsgemäße Dentalmaterial Erdalkali-, Alkali- und/oder Ammonium-Salz der Phosphonsäuren.

Eine bevorzugte Ausgestaltung der Erfindung ist der Zusatz von Mono-, Di- oder Oligomethacrylaten zu dem erfindungsgemäßen Dentalmaterial.

Das Dentalmaterial enthält gemäß einer weiteren bevorzugten Ausführungsform zusätzlich Füllstoffe, bevorzugt ionenfreisetzende Füllstoffe.

Bevorzugt enthält das erfindungsgemäße Dentalmaterial zusätzlich Starter.

Gegenstand der Erfindung ist ferner die Verwendung eines erfindungsgemäßen Dentalmaterials als Haftvermittler, Compomer oder polymerisierbarer Zement.

Bei der Verwendung als Haftvermittler kann das Dentalmaterial ausschließlich aus den genannten Phosphonsäuren bzw. deren Salzen bestehen, es kann ein oder mehrere Lösungsmittel enthalten und/oder zusätzliche polymerisierbare Monomere wie insbesondere Acrylate oder Methacrylate.

Geeignete Lösungsmittel für Dentalmaterialien sind dem Fachmann geläufig, bevorzugt werden Wasser, Methanol, Ethanol, Isopropanol, Aceton, Ethylmethylketon, Ethylacetat sowie Mischungen der vorgenannten Stoffe.

Ein Zusatz wasserlöslicher Methacrylate wie z. B. Hydroxyethyl(meth)acrylate oder Hydroxypropyl(meth)acrylate ist bevorzugt. Als weitere (Meth)acrylate eignen sich bevorzugt (Meth)acrylate, die mindestens zwei Methacrylatgruppen aufweisen wie z. B. Ethylenglycoldi(meth)acrylat, Diethylenglycoldi(meth)acrylat, Triethylenglycoldi(meth)acrylat, Tetraethylenglycoldi(meth)acrylat, Hexandioldi(meth)acrylat, Butandioldi(meth)acrylat, Dodecandioldi(meth)acrylat, 2,2-Bis[p-(hydroxy(meth)acryloyloxy)phenyl]propan, ethoxyliertes Bisphenol-A-di(meth)acrylat, Glycerindi(meth)acrylat, Urethandi(meth)acrylat, Urethanpolyester-di(meth)acrylat, Trimethylolpropantri(meth)acrylat, Dipentaerytrit-penta(meth)-acrylat.

Auch eine weitere Säure in Form einer polymerisierbaren Carbonsäure kann hinzugefügt werden. Beispielhaft seien erwähnt Maleinsäure-mono-2-methacryloyloxyethylester, Phthalsäuremono-2-methacryloyloxyethylester und Trimellitsäure-mono-2-methacryloyloxyethylester.

Wenn im Rahmen der Erfindung von Dentalmaterialien die Rede ist, sind damit alle Materialien gemeint, die im Rahmen von restaurativen oder prothetischen Arbeiten an Zähnen Anwendung finden und die erfindungsgemäßen Phosphonate bzw. deren Salze enthalten. Besonders vorteilhaft sind die erfindungsgemäßen Phosphonsäuren in Haftvermittlern einsetzbar.

Erfindungsgemäße Dentalmaterialien können insbesondere folgende Inhaltsstoffe aufweisen:
- 2,5 bis 60 Gew.-% erfindungsgemäße Phosphonsäuren bzw. deren Salze,
- 5 bis 80 Gew.-% weitere radikalisch polymerisierbare Comonomere,
- 0 bis 80 Gew.-% Lösungsmittel,
- 0 bis 2 Gew.-% radikalische Polymerisationsinitiatoren,
- 0 bis 80 Gew.-% Füllstoffe (abhängig von der vorgesehenen Anwendung als Haftvermittler, Zement oder Compomer).

Für Haftvermittler ist ein Anteil von 0-20 Gew.-% Füllstoffen bevorzugt; Zemente und Compomere sind bevorzugt lösemittelfrei und der Füllstoffanteil liegt bevorzugt zwischen 40 und 80 Gew.-%.

Die Erfindung wird nachfolgend anhand von Ausführungsbeispielen beschrieben. Zunächst wird die teilweise mehrstufige Synthese erfindungsgemäßer Phosphonsäuren beschrieben, anschließend die Zusammensetzung von Ausführungsbeispielen erfindungsgemäßer Haftvermittler.

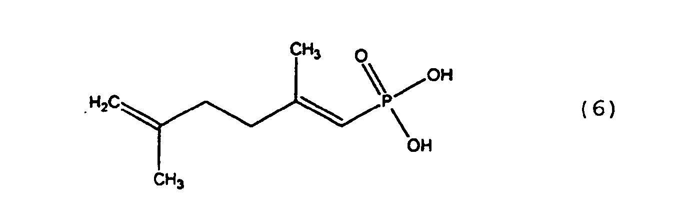

### Synthese von 1-(2,5-Dimethyl-1,5-hexadienyl)-phosphonsäure (Formel (6))

104 g (0,5 mol) PCl₅ werden in 1 1 Toluol suspendiert und unter Kühlen 120 ml (0,6 mol) 2,5-Dimethyl-1,5-hexadien hinzugetropft. Man hält die Reaktion noch 3 h auf 15°C und leitet dann 3 h SO₂ bei 15°C ein. Dann werden Toluol und SOCl₂ abdestilliert, 0,5 g Triphenylphosphan hinzugefügt und die Mischung bei 180°C 8 h bei leichtem Unterdruck gehalten.
Die Mischung wird mit 100 ml Dichlormethan verdünnt und unter Kühlen und heftigem Rühren mit 300 ml einer 5 molaren NaOH-Lösung tropfenweise versetzt. Nach 2 h wird die Reaktionsmischung mit 300 ml Wasser verdünnt und unter Kühlen mit 300 ml 25 %iger Phosphorsäure versetzt.

Diese Mischung wird dreimal mit 500 ml Dichlormethan ausgeschüttelt, die organische Phase über MgSO₄ getrocknet und im Vakuum das Lösungsmittel entfernt. Es entstehen 42 g eines braunen Sirups.
Ausbeute: 44 %

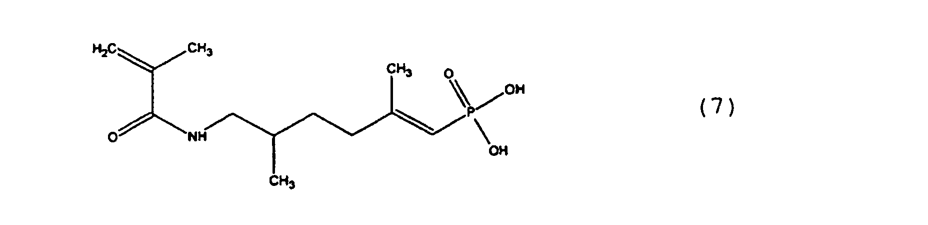

### Synthese von 6-Methacrylamido-2,5-dimethyl-1-hexenylphosphonsäure (Formel (7))

19 g 1-(2,5-Dimethyl-1,5-hexadienyl)-phosphonsäure (0,1 mol) werden in 100 ml Eisessig gelöst und mit 60 mg Phenothiazin und 7 g (0,1 mol) Methacrylnitril versetzt. Unter Kühlen werden 12 g 85 %iger Schwefelsäure hinzugefügt. Nach 24 h bei 50 °C wird der Ansatz gekühlt und mit 200 ml Wasser und 200 ml Dichlormethan versetzt und insgesamt dreimal mit 200 ml Dichlormethan ausgeschüttelt. Die vereinigten organischen Phasen werden noch zweimal mit Wasser ausgeschüttelt und dann über Molekularsieb getrocknet.
Ausbeute: 18 g (66 %)

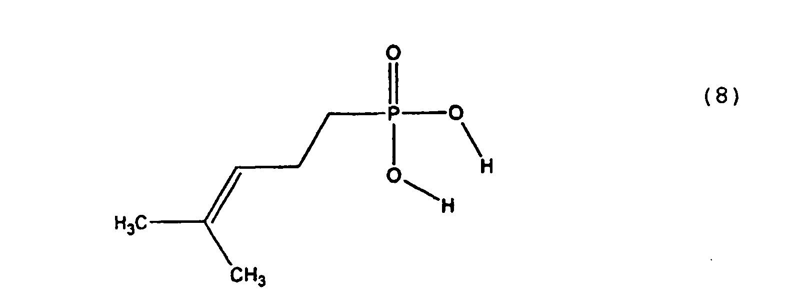

### Synthese von 1-(4-Methyl-4-pentenyl)-phosphonsäure (Formel (8))

4 g 1-Brom-4-methyl-4-penten (48 mmol) werden 6 h mit 7,5 g (60 mmol) P(OCH₃)₃ bei 120°C unter Stickstoff erhitzt. Danach wird die Lösung auf Raumtemperatur gebracht und mit 35 ml 37 %iger Salzsäure versetzt und nochmals 20 h auf 100°C erhitzt. Danach wird die Lösung wieder abgekühlt und mit 10 ml Wasser versetzt. Die wässrige Phase wird isoliert, es bildet sich ein leicht bräunlicher Niederschlag, der an der Luft getrocknet wird.
Ausbeute: 5,6 g 71 %

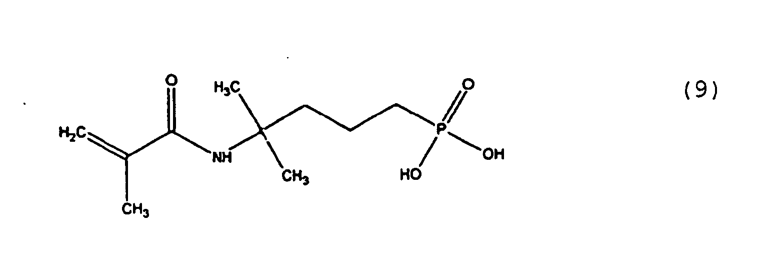

### Synthese von 4-Methacrylamido-4-methyl-pentyl-phosphonsäure (Formel (9))

1,9 g 1-(4-Methyl-4-pentenyl)-phosphonsäure (12) (10 mmol) werden in 10 ml Eisessig gelöst und mit 6 mg Phenothiazin und 0,7 g (10 mmol) Methacrylnitril versetzt. Unter Kühlen werden 1,2 g 85 %iger Schwefelsäure hinzugefügt. Nach 24 h bei 50°C wird der Ansatz gekühlt und mit 20 ml Wasser und 20 ml Dichlormethan versetzt und insgesamt dreimal mit 20 ml Dichlormethan ausgeschüttelt. Die vereinigten organischen Phasen werden noch zweimal mit Wasser ausgeschüttelt und dann über Molekularsieb getrocknet.
Ausbeute: 1,3 g (52 %)

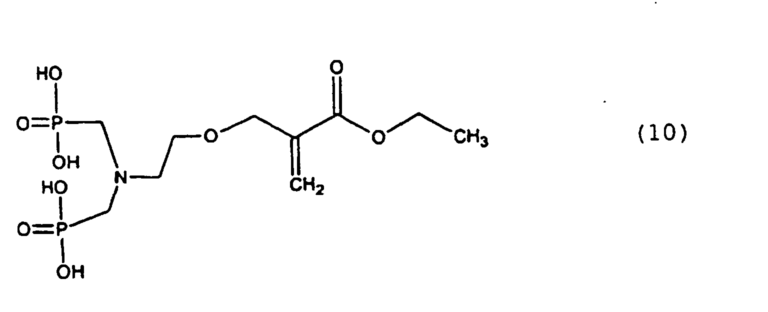

### Synthese von 2-(Ethyloxycarbonyl)-2-propenyl-1-oxyethylaminobismethylenphosphonsäure (Formel (10))

4,45 g (60 mmol) KOH werden in 50 ml DMSO fünf Minuten bei Raumtemperatur gerührt. Dann werden 2,49 g (10 mmol) Ethanolamino-N,N-bismethylenphosponsäure vorsichtig zugegeben. Zu der schwach gefärbten Suspension werden langsam 2,94 g (15 mmol) α-Brommethylacrylsäureethylester zugetropft. Der Reaktionsansatz wird 24 h bei Raumtemperatur gerührt, anschließend mit 50 ml kaltem Wasser versetzt, auf einen sauren pH eingestellt, mit 50 ml Dichlormethan extrahiert, die organische Phase getrocknet und das Lösungsmittel im Vakuum abgetrennt. Der verbleibende wachsartige Rückstand wird im Trokkenschrank getrocknet.
Ausbeute: 1,5 g (40 %)

### Alternative Möglichkeit zur Synthese von 4-Methacrylamido-4-methyl-pentyl-phosphonsäure (Formel (9))

### 1-(4-Methyl-4-pentenyl)-phosphonsäure (Formel (8))

### 1-(4-Methyl-4-pentenyl)-phosphonsäurediethylester

10 ml (0,0752 mol) 5-Brom-2-methyl-2-penten werden mit 15 ml (0,0856 mol) P(OC₂H₅)₃ bei 160 - 190 °C unter Abdestillation des entstehenden Ethylbromids erhitzt. Das Rohprodukt wird weiter verwendet.

### 1-(4-Methyl-4-pentenyl)-phosphonsäure (Formel (8))

4 g (0,0182 mol) 1-(4-Methyl-4-pentenyl)-phosphonsäurediethylester wurden in 40 ml getrocknetem Chloroform gelöst und mit 4 ml (0,04 mol) Trimethylsilybromid versetzt sowie 2 h bei Raumtemperatur gerührt. Anschließend wurde im Vakuum eingeengt und mit 40 ml Ethanol/Wasser (1:1) versetzt und ebenfalls 2 h gerührt. Dann wurde am Rotationsverdampfer eingeengt und der Rückstand im Vakuum mit P₂O₅ getrocknet.

### 4-Methacrylamido-4-methyl-pentyl-phosphonsäure (Formel (9))

2,99 g 1-(4-Methyl-4-pentenyl)-phosphonsäure (0,0182 mol) werden mit 3 mg BHT und 1,53 ml (1,22 g; 0,0182 mol) Methacrylnitril versetzt. Dann wird eine Mischung aus 1 ml (1,84 g 97%iger) Schwefelsäure und 0,32 ml Wasser bei 20 °C getropft. Nach 16 h bei 20 - 30 °C steigert man die Temperatur innerhalb von 4 h auf 60 °C und hält diese Temperatur weitere 16 h. Danach wird der abgekühlte Ansatz in einer Mischung aus 1,16 ml Wasser und 10 ml Methanol gelöst und die Schwefelsäure durch Zugabe einer Lösung aus 1,46 g NaOH, 1,1 ml Wasser und 10 ml Methanol neutralisiert. Das Gemisch muß am Ende aber noch einen sauren pH behalten. Das Natriumsulfat wird abfiltriert und das Filtrat vollständig im Vakuum eingeengt.

Die nachfolgende Tabelle zeigt die Zusammensetzung von drei erfindungsgemäßen Haftvermittlern (Beispiele 1 - 3)

| | **Beispiel 1** | **Beispiel 2** | **Beispiel 3** |
|---|---|---|---|
| Phosphonat | 10 g Formel (7) | 1 g Formel (9) | 1 g Formel (10) |
| H₂O | 5 g | 1 g | 1,5 g |
| Ethanol | 20 g | 4,5 g | 10 g |
| Bis-GMA* | 25 g | 0,75 g | 1 g |
| TEDMA** | 25 g | 1,25 g | 5 g |
| HEMA*** | 15 g | 1,5 g | 1,5 g |
| Ethyl-dimethylaminobenzoat | 0,15 g | 0,018 g | 0,018 g |
| Campherchinon | 0,1 g | 0,014 g | 0,014 g |

| | | | |
|---|---|---|---|
| * Isopropyliden bis[2-hydroxy-3-(4-phenoxy)-methacrylat] | | | |
| ** Triethylenglycoldimethacrylat | | | |
| *** 2-Hydroxyethylmethacrylat | | | |

### Beispiel 4: Zwei-Kompenenten-Haftvermittler-System

Die Zusammensetzung eines Haftvermittlersystems, bestehend aus einem Primer, der als aktive Substanz eines der erfindungsgemäßen Haftmonomere enthält, sowie eines Bonds, das in einem zweiten Schritt auf eine zuvor mit Primer versehene Zahnhartsubstanz aufgetragen wird, ist im nachfolgenden zusammengestellt:

### Komponente 1: Primer

| Substanz | Anteil / Gew.-% |
|---|---|
| 4-Methacrylamido-4-methylpentylphosphonsäure (Formel (9)) | 40,000 |
| Wasser | 28,875 |
| Ethanol | 28,875 |
| Campherchinon | 0,800 |
| 2-Ethylhexyl-4-(dimethylamino)benzoat | 1,400 |
| 2,6-Di tert.butyl-4-methylphenol | 0,050 |

Die einzelnen Substanzen des Primers werden in einem Glasgefäß eingewogen und unter Rühren bei Raumtemperatur vermischt bis eine homogene Lösung entstanden ist.

### Komponente 2: Bond

Als Bond findet der kommerziell erhältliche Primer B des Haftvermittlersystems Ecusit - Primer / Mono (Dental Material Gesellschaft mbH - Hamburg, Deutschland) Verwendung.

### Beispiel 5: Ein-Komponenten-Haftvermittler-System

Die Zusammensetzung eines Haftvermittlersystems, bestehend aus einer Komponente, die u.a. eines der erfindungsgemäßen Haftmonomere enthält, ist in der nachfolgenden Tabelle zusammengestellt.

| Substanz | Anteil / Gew.-% |
|---|---|
| 4-Methacrylamido-4-methylpentylphosphonsäure (Formel (9)) | 10,00 |
| Wasser | 10,00 |
| Ethanol | 44,28 |
| Glycidyldimethacrylat | 10,97 |
| Hydroxyethylmethacrylat | 15,00 |
| Bis-GMA | 7,50 |
| Campherchinon | 0,80 |
| 2-Ethylhexyl-4-(dimethylamino)benzoat | 1,40 |
| 2,6-Di tert.butyl-4-methylphenol | 0,05 |

Die einzelnen Substanzen des Haftvermittlers werden in einem jeweiligen Glasgefäß eingewogen und unter Rühren bei Raumtemperatur vermischt bis eine homogene Lösung entstanden ist.

### Beispiel 6 (Vergleichsbeispiel):

Das Bond aus Beispiel 4 (Primer B des Ecusit Primer/Mono-Haftvermittler-Systems, Dental Material Gesellschaft, Hamburg, Deutschland) wird ohne vorherige Verwendung des Primers aus Beispiel 4 verwendet.

### Shear Bond Strength Messung:

Zur Messung der Haftung von Kompositen an Zahnhartsubstanz unter Verwendung der erfindungsgemäßen Haftvermittler wird ein Shear-Bond-Versuch durchgeführt. Hierzu werden Rinder-Schneidezähne, aus denen zuvor die Pulpa entfernt wurde und die nachträglich in 0,5 Gew.-% Chloramin T-Lösung in Wasser gelagert wurden, auf der Vorderseite bis zum Dentin naß abgeschliffen und anschließend mit einem feinen Schleifpapier (P500) naß plan geschliffen. Nach kurzer Lagerung in demineralisiertem Wasser wird die beschliffene Oberfläche trocken geblasen und das Haftvermittlersystem aufgetragen.

### Zwei-Komponenten-Haftvermittler (Beispiel 4)

Der Primer aus Beispiel 4 wird mit einem Micro-Pinsel 20 s in die beschliffene Dentin-Oberfläche einmassiert. Im Anschluß wird mit dem Bond aus Beispiel 4 analog verfahren, die Oberfläche wird verblasen und 20 s mit einer Dentallampe (Translux EC, Fa. Kulzer & Co GmbH, Wehrheim, Deutschland) belichtet. Eine zweiteilige Teflonform mit einer Kavität von 3,0 mm Durchmesser wird aufgesetzt, mit einem Dental-Komposit (Ecusit, Dental Material Gesellschaft mbH, Hamburg, Deutschland) gefüllt und 40 s belichtet (Kulzer Translux EC).

### Ein-Komponenten-Haftvermittler (Beispiel 5)

Der Haftvermittler aus Beispiel 5 wird mit einem Micro-Pinsel 20 s in die beschliffene Dentin-Oberfläche einmassiert. Im Anschluß wird die Oberfläche verblasen und 20 s mit einer Dentallampe (Translux EC, Fa. Kulzer & Co GmbH, Wehrheim, Deutschland) belichtet. Der Haftvermittler aus Beispiel 5 wird nochmals mit einem Micro-Pinsel 20 s in die Dentin-Oberfläche einmassiert, die Oberfläche verblasen und 20 s mit einer Dentallampe (Translux EC, Fa. Kulzer & Co GmbH, Wehrheim, Deutschland) belichtet. Eine zweiteilige Teflonform mit einer Kavität von 3,0 mm Durchmesser wird aufgesetzt, mit einem Dental-Komposit (Ecusit, Dental Material Gesellschaft mbH, Hamburg, Deutschland) gefüllt und 40 s belichtet (Kulzer Translux EC).

### Vergleichsbeispiel (Beispiel 6)

Das Bond aus Beispiel 6 wird mit einem Micro-Pinsel 20 s in die beschliffene Dentin-Oberfläche einmassiert, mit Druckluft verblasen und 20 s mit einer Dentallampe (Translux EC, Fa. Kulzer & Co GmbH, Wehrheim, Deutschland) belichtet. Eine zweiteilige Teflonform mit einer Kavität von 3,0 mm Durchmesser wird aufgesetzt, mit einem Dental-Komposit (Ecusit, Dental Material Gesellschaft mbH, Hamburg, Deutschland) gefüllt und 40 s belichtet (Kulzer Translux EC).

Die Teflonform wird jeweils entfernt und die Präparation in Wasser zunächst 23 h bei 37°C dann 1 h bei 23°C gelagert. Die Präparation wird anschließend in eine Halterung zur Messung der Shear-Bond-Strength (s. ISO/DTS Committee Draft 11405, Ref. Nr. ISO/TC 106/SC 1 N 321 und dort zitierte Literatur) eingespannt und in einer Apparatur zur Bestimmung eines Kraft-Weg-Diagramms (Z010, Zwick GmbH & Co, Ulm, Deutschland) bei einem Vorschub von 0,50 mm/min vermessen. Die Prüfung wird an mehreren Präparationen (Anzahl n) durchgeführt.

Die Ergebnisse der Untersuchungen sind in der nachfolgenden Tabelle zusammengefaßt:

| Beispiel | n | Scherfestigkeit Mittelwert [MPa] |
|---|---|---|
| 4 | 10 | 17 |
| 5 | 3 | 9 |
| 6 | 10 | 0¹⁾ |

| | | |
|---|---|---|
| ¹⁾Komposit-Prüfkörper lösen sich ohne Widerstand von der Dentin-Oberfläche ab. | | |

## Patentansprüche

1. Dentalmaterial, **dadurch gekennzeichnet, daß** es eine oder mehrere Phosphonsäuren folgender Struktur und/oder Salze dieser Säuren enthält: 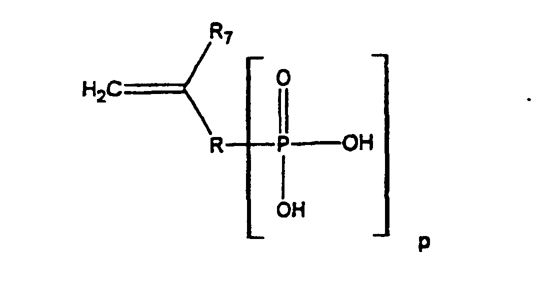 wobei R, R₇ und p unabhängig voneinander bedeuten:
entweder:
a) R: Alkylgruppe oder Alkylengruppe mit mindestens 6 C-Atomen oder Arylgruppe,
R₇: H, Methyl, Ethyl, Propyl, Isopropyl oder Butyl, p gleich 1 oder 2;
oder:
b) R: -CO-NR₁-R₈-
mit R₁ gleich H, Alkyl oder Aryl,
mit R₈ gleich Aryl oder CₙH₂ₙ
wobei 4 ≤ n ≤ 18 ist,
oder R₈ gleich CₙH₂ₙ-Si(R₅)₂-[O-Si(R₅)₂]ₘ-CₙH₂ₙ-
wobei 3 ≤ n ≤ 12,
1 ≤ m ≤ 10, und
R₅ gleich Methyl, Ethyl oder Phenyl ist,
oder R₈ gleich CₙH₂ₙ-COONH-CₙH₂ₙ
wobei 4 ≤ n ≤ 12 ist, und wobei R₈ Ether- oder weitere Urethangruppen aufweisen kann,
R₇ und p wie in a) definiert sind;
oder:
c) R: 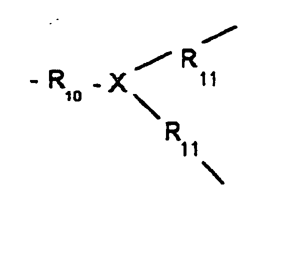 mit R₁₀ ausgewählt aus der Gruppe bestehend aus Arylgruppen, Alkylgruppen mit mindestens 3 C-Atomen, oder Polyethergruppen mit 1 bis 10 Polyethereinheiten, mit R₁₁ gleich oder verschieden ausgewählt aus der Gruppe bestehend aus Alkylgruppen und Arylgruppen, mit X gleich N, B oder CH,
R₇: COOR₉, CONHR₉, H oder Phenyl mit R₉ ausgewählt aus der Gruppe bestehend aus Methyl, Ethyl, Propyl, Isopropyl und Butyl,
p gleich 2.

2. Dentalmaterial nach Anspruch 1, **dadurch gekennzeichnet, daß** die Phosphonsäuren folgende Struktur aufweisen: 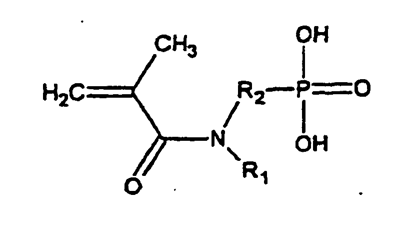 wobei R₂ eine Alkylgruppe mit mindestens 4, vorzugsweise 6 bis 12 C-Atomen oder eine Arylgruppe und R₁ gleich Alkyl, Aryl oder H ist.

3. Dentalmaterial nach Anspruch 1, **dadurch gekennzeichnet, daß** die Phosphonsäuren folgende Struktur aufweisen: 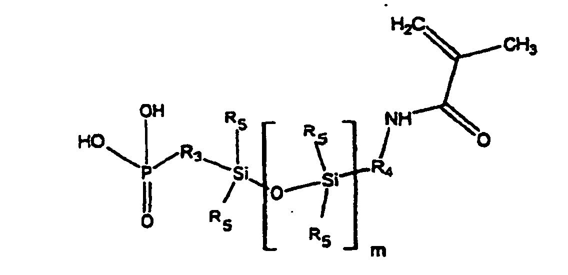 wobei R₃, R₄ gleich oder verschieden Aryl oder Alkyl darstellt, R₅ gleich oder verschieden Methyl, Ethyl oder Phenyl darstellt, und m gleich 1 bis 10 ist.

4. Dentalmaterial nach Anspruch 1, **dadurch gekennzeichnet, daß** die Phosphonsäuren folgende Struktur aufweisen: 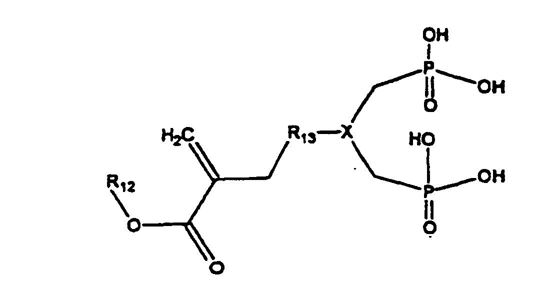 wobei bedeuten:
X gleich N, B, oder CH,
R₁₂ gleich H, Methyl, Ethyl, Propyl, iso-Propyl oder Butyl,
R₁₃ gleich Alkyl mit mindestens 3 C-Atomen Kettenlänge, Aryl oder eine Polyethergruppe mit 1 bis 10 PolyetherEinheiten.

5. Dentalmaterial nach einem der Ansprüche 1 bis 4, , **dadurch gekennzeichnet, daß** es zusätzlich Lösungsmittel enthält.

6. Dentalmaterial nach Anspruch 5, **dadurch gekennzeichnet, daß** das Lösungsmittel ausgewählt ist aus der Gruppe bestehend aus Wasser, Ethanol, Methanol, Isopropanol, Aceton, Ethylmethylketon und Ethylacetat.

7. Dentalmaterial nach einem der Ansprüche 1 bis 6, **dadurch gekennzeichnet, daß** es Erdalkali-, Alkali- und/oder Ammonium-Salz der Phosphonsäuren enthält.

8. Dentalmaterial nach einem der Ansprüche 1 bis 7, **dadurch gekennzeichnet, daß** es zusätzlich Mono-, Di- oder Oligomethacrylate enthält.

9. Dentalmaterial nach einem der Ansprüche 1 bis 8, **dadurch gekennzeichnet, daß** es zusätzlich Füllstoffe enthält.

10. Dentalmaterial nach Anspruch 9, **dadurch gekennzeichnet, daß** die Füllstoffe ionenfreisetzend sind.

11. Dentalmaterial nach einem der Ansprüche 1 bis 10, **dadurch gekennzeichnet, daß** es zusätzlich Starter enthält.

12. Verwendung eines Dentalmaterials nach einem der Ansprüche 1 bis 11 als Haftvermittler oder Bestandteil eines Haftvermittlers.

13. Verwendung eines Dentalmaterials nach einem der Ansprüche 1 bis 11 als Compomer oder polymerisierbarer Zement oder als Bestandteil eines solchen Compomers bzw. Zements.

## Claims

1. A dental material, **characterized in that** it comprises one or more phosphonic acids with the following structure and/or salts of these acids: 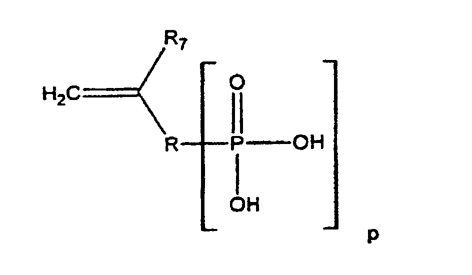 in which R, R₇ and p are, independently of one another:
either:
a) R: alkyl group or alkylene group with at least 6 C-atoms or aryl group,
R₇: H, methyl, ethyl, propyl, isopropyl or butyl,
p is 1 or 2;
or:
b) R: -CO-NR₁-R₈-
with R₁ equal to H, alkyl or aryl,
with R₈ equal to aryl or CₙH₂ₙ
in which 4 ≤ n ≤ 18,
or R₈ is CₙH₂ₙ-Si(R₅)₂-[O-Si(R₅)₂]ₘ-CₙH₂ₙ
in which 3 ≤ n ≤ 12,
1 ≤ m ≤ 10, and
R₅ is methyl, ethyl or phenyl,
or R₈ is CₙH₂ₙ-COONH-CₙH₂ₙ
in which 4 ≤ n ≤ 12, and in which R₈ can have ether or additional urethane groups,
R₇ and p are defined as in a);
or:
c) R: 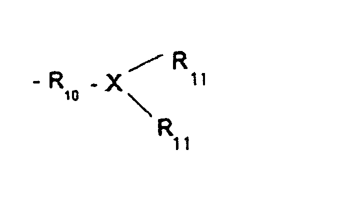 with R₁₀ chosen from the group consisting of aryl groups, alkyl groups with at least 3 C-atoms or polyether groups with 1 to 10 polyether units and with R₁₁ chosen, identically or differently, from the group consisting of alkyl groups and aryl groups,
with X equal to N, B or CH,
R₇: COOR₉, CONHR₉, H or phenyl with R₉ chosen from the group consisting of methyl, ethyl, propyl, isopropyl and butyl,
p is equal to 2.

2. The dental material as claimed in claim 1, **characterized in that** the phosphonic acids have the following structure: 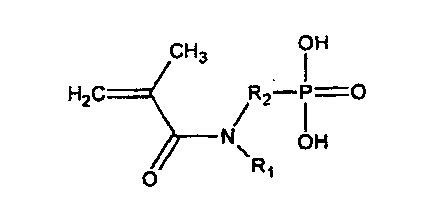 in which R₂ is an alkyl group with at least 4, preferably 6 to 12, C-atoms or an aryl group and R₁ is alkyl, aryl or H.

3. The dental material as claimed in claim 1, **characterized in that** the phosphonic acids have the following structure: 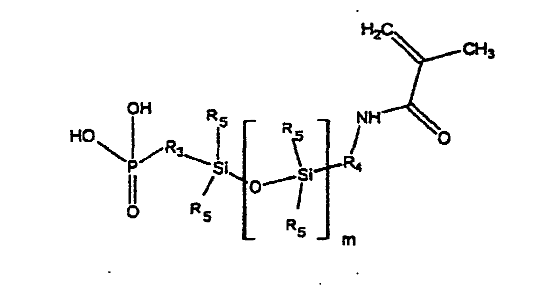 in which R₃ and R₄, which are identical or different, are aryl or alkyl, R₅, which are identical or different, are methyl, ethyl or phenyl, and m is 1 to 10.

4. The dental material as claimed in claim 1, **characterized in that** the phosphonic acids have the following structure: 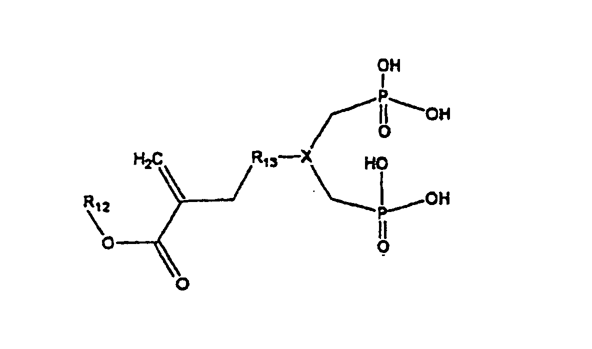 in which:
X is N, B or CH,
R₁₂ is H, methyl, ethyl, propyl, isopropyl or butyl, R₁₃ is alkyl with a chain length of at least 3 C-atoms, aryl or a polyether group with 1 to 10 polyether units.

5. The dental material as claimed in one of claims 1 to 4, **characterized in that** it additionally comprises solvents.

6. The dental material as claimed in claim 5, **characterized in that** the solvent is chosen from the group consisting of water, ethanol, methanol, isopropanol, acetone, ethyl methyl ketone and ethyl acetate.

7. The dental material as claimed in one of claims 1 to 6, **characterized in that** it comprises alkaline earth metal, alkali metal and/or ammonium salts of the phosphonic acids.

8. The dental material as claimed in one of claims 1 to 7, **characterized in that** it additionally comprises mono-, di- or oligomethacrylates.

9. The dental material as claimed in one of claims 1 to 8, **characterized in that** it additionally comprises fillers.

10. The dental material as claimed in claim 9, **characterized in that** the fillers are ion-releasing.

11. The dental material as claimed in one of claims 1 to 10, **characterized in that** it additionally comprises initiator entities.

12. Use of the dental material as claimed in one of claims 1 to 11, as adhesion promoter or component of an adhesion promoter.

13. Use of the dental material as claimed in one of claims 1 to 11, as compomer or polymerizable cement or as component of such a compomer or cement.

## Revendications

1. Matériau dentaire, **caractérisé en ce qu'**il comprend un ou plusieurs acides phosphoniques et/ou sels de ces acides de structure suivante : 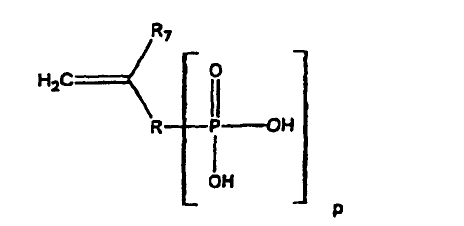 dans laquelle R, R₇ et p indépendamment les uns des autres signifient :
soit :
a) R : groupe alkyle ou alkylène ayant au moins 6 atomes de carbone ou groupe aryle,
R₇ : H, méthyle, éthyle, propyle, isopropyle ou butyle,
p égal à 1 ou 2 ;
soit :
b) R : -CO-NR₁-R₈-
avec R₁ égal à H, alkyle ou aryle,
avec R₈ égal à aryle ou CₙH₂ₙ,
où 4 ≤ n ≤ 18,
ou bien R₈ égal à CₙH₂ₙ-Si(R₅)₂-[O-Si(R₅)₂]ₘ-CₙH₂ₙ-
où 3 ≤ n ≤ 12,
1 ≤ m ≤ 10, et
R₅ égal à méthyle, éthyle ou phényle,
ou bien R₈ égal à CₙH₂ₙ-COONH-CₙH₂ₙ
où 4 ≤ n ≤ 12, et où R₈ peut présenter des groupes éther ou d'autres groupes uréthane,
R₇ et p sont tels que définis dans la section a) ;
soit :
c)R: 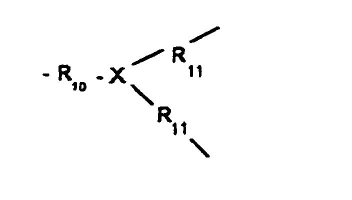 avec Rio choisi dans le groupe constitué des groupes aryle, groupes alkyle ayant au moins 3 atomes de carbone, ou groupes polyéther ayant de 1 à 10 motifs polyéther, avec R₁₁ identiques ou différents choisis dans le groupe constitué des groupes alkyle et aryle, avec X égal à N, B ou CH,
R₇ : COOR₉, CONHR₉, H ou phényle avec R₉ choisi dans le groupe constitué du méthyle, éthyle, propyle, isopropyle et butyle,
p égal à 2.

2. Matériau dentaire selon la revendication 1, **caractérisé en ce que** les acides phosphoniques présentent la structure suivante : 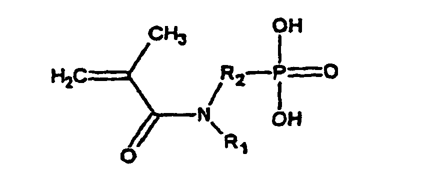 dans laquelle R₂ est un groupe alkyle ayant au moins 4, de préférence 6 à 12 atomes de carbone ou un groupe aryle et Ri égal à alkyle, aryle ou H.

3. Matériau dentaire selon la revendication 1, **caractérisé en ce que** les acides phosphoniques présentent la structure suivante : 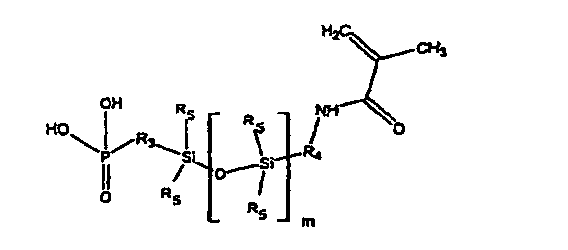 dans laquelle R₃, R₄ identiques ou différents représentent aryle ou alkyle, R₅ identiques ou différents représentent méthyle, éthyle ou phényle, et m est égal à un nombre de 1 à 10.

4. Matériau dentaire selon la revendication 1, **caractérisé en ce que** les acides phosphoniques présentent la structure suivante : 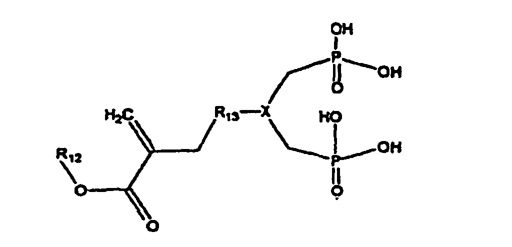 où les symboles signifient :
X égal à N, B, ou CH,
R₁₂ égal à H, méthyle, éthyle, propyle, isopropyle ou butyle,
R₁₃ égal à alkyle avec une longueur de chaîne d'au moins 3 atomes de carbone, aryle ou un groupe polyéther ayant 1 à 10 motifs polyéther.

5. Matériau dentaire selon l'une des revendications 1 à 4, **caractérisé en ce qu'**il comprend en plus des solvants.

6. Matériau dentaire selon la revendication 5, **caractérisé en ce que** le solvant est choisi dans le groupe constitué de l'eau, de l'éthanol, du méthanol, de l'isopropanol, de l'acétone, de l'éthylméthylcétone et de l'acétate d'éthyle.

7. Matériau dentaire selon l'une des revendications 1 à 6, **caractérisé en ce qu'**il comprend un sel alcalino-terreux, alcalin ou d'ammonium des acides phosphoniques.

8. Matériau dentaire selon l'une des revendications 1 à 7, **caractérisé en ce qu'**il comprend en plus des mono-, di- ou oligométhacrylates.

9. Matériau dentaire selon l'une des revendications 1 à 8, **caractérisé en ce qu'**il comprend en plus des charges.

10. Matériau dentaire selon la revendication 9, **caractérisé en ce que** les charges libèrent des ions.

11. Matériau dentaire selon l'une des revendications 1 à 10, **caractérisé en ce qu'**il comprend en plus un starter.

12. Utilisation d'un matériau dentaire, selon l'une des revendications 1 à 11, à titre d'adhésif ou de composant d'un adhésif.

13. Utilisation d'un matériau dentaire, selon l'une des revendications 1 à 11, à titre de compomère ou de ciment polymérisable ou à titre de composant d'un tel compomère ou ciment.
